# EUROPÄISCHE PATENTSCHRIFT

(11) **EP 2 464 249 B1**
(45) Veröffentlichungstag und Bekanntmachung des Hinweises auf die Patenterteilung: **13.03.2013**
(21) Anmeldenummer: 11711330.8
(22) Anmeldetag: 29.03.2011
(51) Int. Cl.: A23L 2/52, A61F 3/00, A61K 36/82, A61P 25/00, A61P 25/28, A61K 36/28, A23L 2/39, A23L 1/30

(54) **KONZENTRATIONSSTEIGERNDES GETRÄNK**
CONCENTRATION-ENHANCING DRINK
BOISSON PERMETTANT D'AUGMENTER LA CONCENTRATION

(30) Priorität: 29.03.2010 EP 10158180
(43) Veröffentlichungstag der Anmeldung: 20.06.2012
(73) Patentinhaber: Huber, Johannes, 1130 Wien (AT); Hochegger, Paul, 1040 Wien (AT)
(72) Erfinder: HUBER, Johannes, 1130 Wien (AT)
(74) Vertreter: Sonn & Partner Patentanwälte
(86) Internationale Anmeldenummer: PCT/EP2011/054825
(87) Internationale Veröffentlichungsnummer: WO 2011/120969

(56) Entgegenhaltungen:
- WO-A2-2008/006082
- JP-A- 11 018 722
- JP-A- 2003 286 167
- US-A1- 2002 086 067
- US-A1- 2008 213 401

## Beschreibung

Die vorliegende Erfindung betrifft konzentrationssteigernde Zusammensetzungen.

Mit "Konzentration" wird die willentliche Fokussierung der Aufmerksamkeit auf eine bestimmte Tätigkeit, das Erreichen eines kurzfristig erreichbaren Ziels oder das Lösen einer gestellten Aufgabe bezeichnet. Dabei wird unter der "willentlichen Fokussierung" verstanden, dass eine Person bewusst für eine gewisse Zeit auf das gerade Ausgeübte (oder Kommende) oder Empfundene achtet. Eine konzentrierte Tätigkeit ist mit geistiger Anstrengung verbunden, wobei jedoch der Grad der Konzentriertheit in der Regel mit der Zeit nachlässt, das "Aufmerksamkeitsniveau" sinkt.

Unter einer "Konzentrationsstörung" wird allgemein eine Beeinträchtigung der Fähigkeit verstanden, die Aufmerksamkeit auf eine bestimmte (geistige) Tätigkeit gerichtet zu halten. Die Konzentrationsstörung wird vor allem in der Medizin, in der Psychotherapie und in der Pädagogik als Symptombeschreibung verwendet.

Konzentration und Konzentrationsstörungen werden in der Regel wissenschaftlich mit standardisierten Tests (Aufmerksamkeits-Belastungs-Tests) gemessen bzw. festgestellt.

Zu den Faktoren, die die Konzentration beeinflussen, gehören der physische und psychische Zustand der jeweiligen Person, insbesondere abhängig von Ernährung, Umgebungsbedingungen und sozialen Komponenten. Konzentrationsstörungen können dabei auf neurologische, psychosomatische oder organische Ursachen zurückzuführen sein.

Im Stand der Technik sind sehr viele Mittel und Wege bekannt, um die Konzentration zu steigern, das Aufmerksamkeitsniveau möglichst lange aufrecht zu erhalten oder Konzentrationsstörungen zu verbessern oder auszuräumen.

So ist bekannt, dass sich Tagschlaf, Entspannung, moderater Ausdauersport oder Konzentrationsübungen positiv auf die Konzentration und die Beibehaltung des Aufmerksamkeitsniveaus auswirken, wohingegen Stress oder Schlafmangel konzentrationsstörend sind. Es ist ebenfalls beschrieben, dass sich ein ausgeglichener Glukosespiegel positiv auf die Konzentration auswirken kann, wohingegen ein sehr niedriger Glukosespiegel die Konzentration erschweren kann.

Es sind auch zahlreiche flüssige und feste Lebensmittel beschrieben, denen man konzentrationssteigernde Wirkungen nachsagt, z.B. Omega-3-Fettsäuren (Fischöl), Vitamine, vor allem Vitamin B, Lentaya, Koffein, Ginseng, Guarana, Ginkgo, Lecitin, Curcumin, etc..

Aufgabe der vorliegenden Erfindung ist, ein konzentrationssteigerndes Getränk zur Verfügung zu stellen.

Demgemäß betrifft die vorliegende Erfindung ein Getränk, enthaltend jeweils unabhängig voneinander pro 1
- 1 mg bis 10 g, vorzugsweise 1 bis 1000 mg, noch bevorzugter -10 bis 500 mg, insbesondere 50 bis 300 mg, Epigallokatechingallat (EGCG),
- 1 bis 500 mg, vorzugsweise 10 bis 100 mg, insbesondere 20 bis 80 mg, Nikotinamid-Ribose (NR) und/oder 0,05 bis 10 g, vorzugsweise 0,1 bis 5 g, insbesondere 0,5 bis 3 g, Tryptophan.

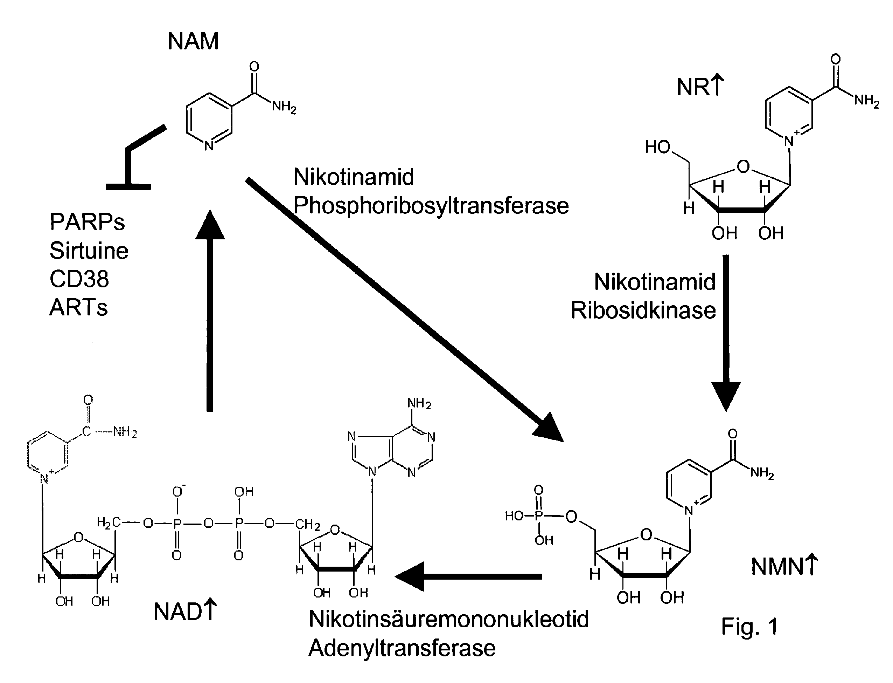

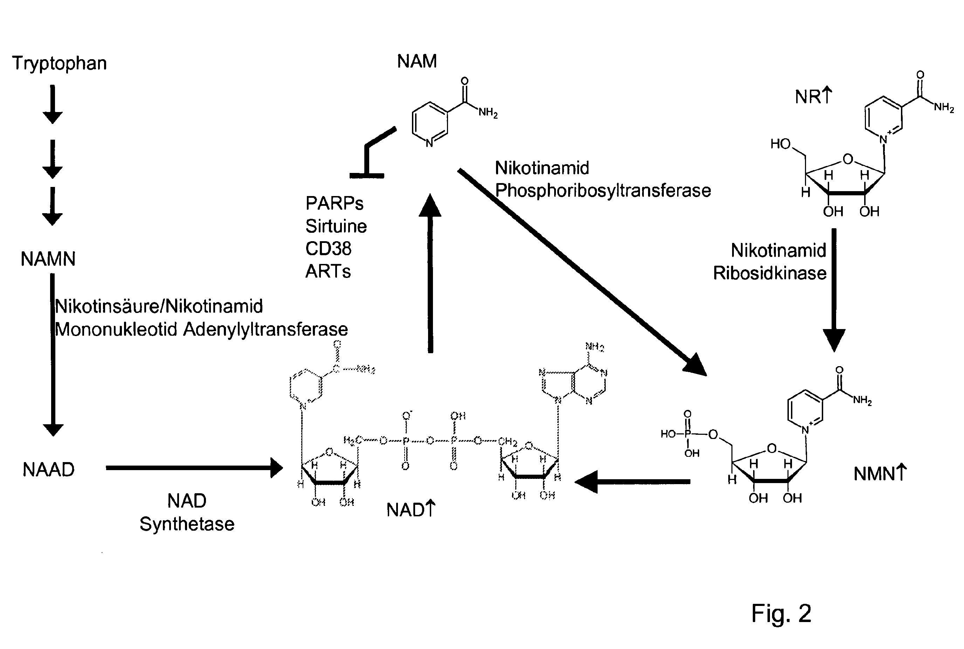

Epigallokatechingallat (EGCG; (2R,3R)-2-(3,4,5-Trihydroxyphenyl)-3,4-dihydro-1(2H)-benzopyran-3,5,7-triol-3-(3,4,5-trihydroxybenzoat)) ist ein natürlich vorkommendes Antioxidans. EGCG ist ein Katechin, das zur Untergruppe der Polyphenole zählt und in grünem Tee vorkommt. Neben vielen anderen Wirkungen weist EGCG einen Insulin-sedierenden Effekt sowie eine konzentrationssteigernde Wirkung auf. EGCG vermehrt die endogene Bildung des Nikotinsäureamid-Adenin-Dinukleotids (NAD (= NAD⁺)). Durch Gabe von EGCG wird die Nikotinamid-Ribosid-Kinase angeregt und vermehrt Beta-Nikotinamid-Mononukleotid gebildet. Das mit der Diät aufgenommene Tryptophan wird in der Zelle in Nikotinsäure-Mononukleotid (NAMN) umgewandelt, welches wieder durch Nikotinsäure/Nikotinamid-Mononukleotid Adenyltransferase in Nikotinsäure-Adenin-Dinukleotid (NAAD) umgewandelt wird, aus dem über die NAD Synthase das NAD entsteht. EGCG regt die NAD-Synthase sowie die Nikotinsäure/Nikotinamid Mononukleotid Adenyltransferase an, wodurch bei gleichzeitigem Vorhandensein des Tryptophans die endogene Bildung von NAD erhöht wird (siehe Fig. 1 und 2). EGCG wird vorzugsweise als Extrakt aus biologischen Quellen im erfindungsgemäßen Getränk vorgesehen, besonders bevorzugt als Extrakt aus Teepflanzen, insbesondere direkt aus dem grünen Tee oder nach dem Matcha-Verfahren gewonnen.

Mittel, welche EGCG entweder als Reinsubstanz oder als Bestandteil von grünem Tee enthalten und welche u.a. zur Behandlung von mentalen Krankheiten oder Befindlichkeitsstörungen eingesetzt werden, sind u.a. in JP 203/286167 A, JP 11/018722 A, US 2002/086067 A1, US 2008/213401 A1 und WO 2008/006082 A2 beschrieben.

Auch die Nikotinamid-Ribose (NR; Nikotinamid-Ribosid) (1-[3,4-Dihydroxy-5-(hydroxymethyl)oxolan-2-yl]pyridin-5-carboxamid; auch als "Nikotinamid-Ribosid", "Nikotinamid-beta-Ribosid", "Nikotinamid-Ribonukleosid", "1-[(2R,3R,4S,5R)-3,4-Dihydroxy-5-(hydroxymethyl)oxolan-2-yl]pyridin-5-carboxamid", "N-Ribosylnikotinamid" bezeichnet (CAS: 1341-23-7)) ist ein Präkursor für das NAD und wird durch die Nikotinamid-Ribose-Kinase (nrk) in Nikotinamid-Mononukleotid umgewandelt, welches seinerseits über Nikotinamid Phosphoribosyltransferase (NNAD) in NAD konvertiert wird. Sowohl die Nikotinamid-Ribose-Kinase wie auch die Nikotinsäure-Nikotinamid-Mononukleotid-Adenyltransferase werden durch EGCG stimuliert, sodass die gleichzeitige Verabreichung von entweder Tryptophan oder Nikotinamid-Ribose mit EGCG zu einer verstärkten endogenen NAD-Synthase führt. Gleichzeitig wird dadurch auch die Aktivität der Sirtuine, die NAD abhängig ist, angeregt. Damit ist die Aktivität der Nikotinamid-Ribosidkinase mit der Aktivität der Sirtuine (vor allem Sirt 2) assoziiert. Im Zuge der vorliegenden Erfindung konnten auch Erkenntnisse über NR bei der zellulären NAD-Herstellung gewonnen werden, die NR als eine der zentralen Verbindungen in dieser Synthese ausweisen.

Tryptophan ((S)-2-Amino-3-(1H-indol-3-yl)propansäure) ist eine für den Menschen essentielle Aminosäure.

Wie EGCG können auch NR und Tryptophan sowohl in Form natürlicher Extrakte als auch in synthetischer Form (vgl. z.B. Yang et al., J. Med. Chem. 50 (2007): 6458 - 6461) erfindungsgemäß verwendet werden; eine andere vorteilhafte Variante ist die enzymatische Herstellung der Komponenten, insbesondere von NR. Bevorzugt sind in allen Fällen (z.B. aus natürlichen Quellen, aus Präparationen chemischer Synthesen oder Produkten aus enzymatischen Synthesen (oder Kombinationen derselben)) aufgereinigte Präparationen als Ausgangsstoffe zur Herstellung des erfindungsgemäßen Getränkes (z.B. solche Extrakte oder Präparate, die EGCG und NR und/oder Tryptophan zu zumindest 10 Gew.-% enthalten, vorzugsweise zu zumindest 30 Gew.-%, insbesondere zu zumindest 50 Gew.-%, jeweils bezogen auf Trockengewicht). Beispielsweise kommt das NR in manchen Nahrungsmitteln, vor allem in der Milch, vor, kann aber auch als diätetisches Nahrungsmittel zugeführt werden. Alle hierin gemachten Mengenangaben oder Prozentanteile beziehen sich auf die jeweiligen Verbindungen in Reinform in der jeweiligen Zusammensetzung (solange nicht explizit etwas Anderes angegeben ist).

Die vorliegende Erfindung basiert auf der konzentrationssteigernden Wirkung einer Kombination aus einer effektiven Menge an EGCG auf der einen Seite und einer effektiven Menge an NR oder an Tryptophan oder einer Kombination aus NR und Tryptophan auf der anderen Seite zur Bereitstellung eines Mittels zur Konzentrationssteigerung. Die erfindungsgemäß erzielte Konzentrationssteigerung kann sich z.B. als Steigerung des relativen Konzentrationsvermögens einer einzelnen Person (gegenüber dem Konzentrationsvermögen derselben Person vor der Einnahme des erfindungsgemäßen Mittels) oder als Verbesserung der geistigen Ausdauer (bzw. Verzögerung des Konzentrationsabfalls bei fortgesetzten konzentrierten Tätigkeiten) ergeben. Demgemäß ist die vorliegende Erfindung vor allem für die Konzentrationssteigerung bei geistigen Tätigkeiten geeignet, insbesondere bei länger andauernden Tätigkeiten, die über längere Zeit ein hohes Konzentrationsvermögen erfordern. Besonders gut einsetzbar ist das erfindungsgemäße Mittel bei Lern- und Ausbildungstätigkeiten (also für Schüler, Studenten, Lehrer), aber auch für spezialisierte Berufe, wie Flug- und Verkehrsüberwachung, Qualitätskontrolle, Bildschirmarbeit, etc.. Auch für Freizeittätigkeiten, die ein bestimmtes Maß an Konzentration erfordern, z.B. für konzentrationsintensive Spiele (Schach, Computerspiele, etc.), ist die vorliegende Erfindung gut nutzbar. Mit der vorliegenden Erfindung kann nicht nur die individuelle Konzentrationsfähigkeit gesteigert werden, es kann auch ein Leistungsabfall bei andauernden Tätigkeiten verzögert oder verhindert werden bzw. dieser Leistungsabfall jedenfalls im Ausmaß signifikant reduziert werden. Die Erfindung ist natürlich primär für die Anwendung beim Menschen gedacht, prinzipiell ist aber auch die Anwendung bei Tieren, z.B. höheren Säugetieren, möglich, da hier dieselben Voraussetzungen bei der synergistischen Stoffwechselfunktion der erfindungsgemäßen Kombination gegeben sind.

Mit der Kombination von EGCG und NR und/oder Tryptophan wird erfindungsgemäß eine Zusammensetzung zur Verfügung gestellt, die konzentrationssteigernde Wirkung aufweist. Mit der erfindungsgemäßen Kombination kann auch das Konzentrationsniveau verlängert werden. Weiters können mit dem erfindungsgemäßen Getränk auch Konzentrationsstörungen verbessert oder sogar überwunden werden.

Vorzugsweise wird dem erfindungsgemäßen Getränk auch ein Süßstoff zugesetzt. Eine bevorzugte Variante enthält dabei den Süßstoff Steviosid (19-O-β-D-Glucopyranosyl-13-O-[β-D-glucopyranosyl(1-2)-β-D-glucopyranosyl]-steviol). Steviosid ist ein Diterpenglykosid und dreihundertmal süßer als Zucker. Steviosid kann aus Steviablättern (Stevia rebaudiana) gewonnen werden und ist für Diabetiker geeignet. Beim erfindungsgemäßen Getränk wird als Süßstoff vorzugsweise Stevia (rebaudiana) oder ein Steviaextrakt zugesetzt, welcher enzymatisch (fermentativ) behandelt sein kann.

Im erfindungsgemäßen Getränk werden vorzugsweise 1 bis 300 mg/l Steviosid vorgesehen. Steviablätter enthalten acht HauptGlykoside (Steviosid, Steviolbiosid, Rebaudiosid A, C, D, E und F sowie Dulcosid A), wobei Steviosid mit sechs bis achtzehn Prozent den größten Anteil an den in Steviablättern gefundenen Wirkstoffen hat. Vier dieser Steviol-Glykoside (Steviosid, Rebaudiosid A, Rebaudiosid C und Dulcosid) sind im Wesentlichen für die Süßwirkung verantwortlich; wovon Rebaudiosid A die besten sensorischen Eigenschaften aller vier Hauptglykoside aufweist, da es am süßesten und wenig bitter ist. Werden enzymatisch gewonnene Steviaprodukte erfindungsgemäß als Süßstoffe vorgesehen, so enthalten sie nahezu 100 % Rebaudioside und nur in geringen Spuren Steviosid. Vorteil dabei ist, dass das Getränk dann keinen bitteren Bei- oder Nachgeschmack aufweist. Rebaudiosid A wird als Süßstoff im erfindungsgemäßen Getränk vorzugsweise von 0,5 mg bis 200 mg vorgesehen (z.B. wenn enzymatisch gewonnene Stevia-Produkte zur Süßung verwendet werden). Hingegen ist bevorzugt, dass die erfindungsgemäße Zusammensetzung zuckerfrei verabreicht wird, da damit negative Wechselwirkungen mit Zucker (Glukose, Saccharose) verhindert werden.

Vorzugsweise enthält das erfindungsgemäße Getränk weitere Hilfs- und Geschmacksstoffe. Dabei kommen vor allem die dem Fachmann bekannten Geschmacksstoffe aus natürlichen Quellen zum Einsatz, z.B. Fruchtsäfte, Fruchtextrakte, Tees, etc..

Im Zusammenwirken mit EGCG und NR und/oder Tryptophan verhindert Steviosid die hyperinsulinämische Wirkung und unterstützt damit von einer anderen Seite den Effekt von EGCG.

Das erfindungsgemäße Getränk kann in allen üblichen Getränkebehältnissen und in marktüblichen Volumina zur Verfügung gestellt werden. Gemäß bevorzugten praktischen Ausführungsformen weisen die erfindungsgemäßen Getränke ein Volumen von 100 ml bis 2 1, vorzugsweise von 250 ml bis 1 1, insbesondere von 330 ml bis 600 ml, auf. Bevorzugte Verkaufsvolumina sind 100, 200, 250, 330 und 500 ml; die 750 ml, 1 l und 2 l Behältnisse sind als Großpackungen geeignet.

Gemäß einer Variante der vorliegenden Erfindung kann das erfindungsgemäße Getränk auch in Trockenform vorgesehen werden, so dass es zur gebrauchsfertigen Form mittels Flüssigkeiten (in der Regel Wasser (insbesondere Mineralwasser), jedoch sind auch Fruchtsäfte, Limonaden und Ähnliches geeignet) rekonstituiert werden kann. Vorzugsweise ist dann die Trockenform in Einzeldosen, beispielsweise als Tagesdosen, abgepackt. Demgemäß beinhalten erfindungsgemäße Tagesdosen (z.B. in Sachets, Brausetabletten, etc.) beispielsweise (auch jeweils unabhängig voneinander) 1 mg bis 10 g, vorzugsweise 1 bis 1000 mg, noch bevorzugter 10 bis 500 mg, insbesondere 50 bis 300 mg, EGCG, 1 bis 500 mg, vorzugsweise 10 bis 100 mg, insbesondere 20 bis 80 mg, NR und/oder 0,05 bis 10 g, vorzugsweise 0,1 1 bis 5 g, insbesondere 0,5 bis 3 g, Tryptophan sowie gegebenenfalls 0,1 mg bis 500 mg, vorzugsweise 0,5 mg bis 200 mg, insbesondere 1 bis 100 mg, Steviosid. Eine derartige Trockenform (also ein Präparat in fester Form, insbesondere in Pulverform), kann auch z.B. in Form von Brausetabletten zur Verfügung gestellt werden. Entsprechende typisch verwendbare Hilfs- und Formulierungsstoffe oder Trägersubstanzen sind dem Fachmann auf diesem Gebiet bekannt und können bei Bedarf in den erfindungsgemäßen Mitteln entsprechend eingesetzt werden. Man kann die erfindungsgemäßen Getränke auch auf Basis höher konzentrierter Konzentrate (z.B. Sirups) zur Verfügung stellen, die dann entsprechend Vorgaben, z.B. 1:2 bis 1:20, vorzugsweise 1:5 bis 1:10, verdünnt werden können, um zu den erfindungsgemäßen Werten der Konzentrationen im Getränk zu gelangen.

Die oben erwähnten Getränke können vorzugsweise auch bereits diese Tagesdosen als Verkaufseinheit enthalten, sodass in einer Verkaufseinheit (100 ml, 200 ml, 250 ml, 330 ml, 500 ml, 750 ml, 1000 ml oder anderen üblichen Größen für Flaschen, Dosen, Getränkepackungen, etc.) die Tagesdosis (beispielsweise (jeweils unabhängig voneinander) 1 mg bis 10 g, vorzugsweise 1 bis 1000 mg, noch bevorzugter 10 bis 500 mg, insbesondere 50 bis 300 mg, EGCG, 1 bis 500 mg, vorzugsweise 10 bis 100 mg, insbesondere 20 bis 80 mg, NR und/oder 0,05 bis 10 g, vorzugsweise 0,1 bis 5 g, insbesondere 0,5 bis 3 g, Tryptophan sowie gegebenenfalls 0,1 mg bis 500 mg, vorzugsweise 0,5 mg bis 200 mg, insbesondere 1 bis 100 mg, Steviosid; neben allfälligen weiteren Inhaltsstoffen) enthalten ist.

Beispielsweise können die erfindungsgemäßen Getränke als Limonaden zur Verfügung gestellt werden. Hierbei können als weitere bevorzugte Zusatzstoffe z.B. Zuckerkulör (bei koffeinhaltigen und den diesen in der Geschmacksrichtung entsprechenden koffeinfreien Limonaden sowie bei Limonaden mit Apfelgeschmack mit oder ohne Fruchtsaftanteil und klaren Kräuterlimonaden), Koffein (bei koffeinhaltigen Limonaden in einem Anteil von mindestens 65 Milligramm pro Liter und höchstens 250 Milligramm pro Liter), Molkenerzeugnisse, Beta-Karotin sowie Riboflavin und färbende Lebensmittel (außer bei klaren Limonaden z.B. mit Zitrus-Aroma), Zitronensäure, Auszüge der Ingwerwurzel (z.B. als Ginger Ale-Produkt) oder Bitterstoffe (z.B. Chinin (aus der Rinde des Chinabaumes; wenn die Limonade mindestens 15 mg/l Chinin enthält, wird sie als Tonic bezeichnet (max. 85 mg/l Chinin))) vorgesehen werden.

Auch eine Kombination mit Fruchtsäften, Gemüsesäften oder Fruchtmark ist eine bevorzugte Variante des erfindungsgemäßen Getränkes. Das fertige Lebensmittel kann dann als Fruchtsaft, Fruchtsaftgetränk, Fruchtnektar, etc. bereitgestellt werden. Weitere bevorzugte Inhaltsstoffe sind die oben angeführten, daneben können z.B. auch Milchsäure, Zitronensäure und Ascorbinsäure zugesetzt werden.

Fruchtsäfte oder deren Bestandteile werden in der Regel durch Erhitzen haltbar gemacht. Ein modernes Verfahren ist die Uperisation (Kunstwort aus "Ultra" und "Pasteurisation"). Auf ca. 80 °C vorgewärmter Saft wird dabei durch Einleiten von Dampf unter Druck für einige Sekunden auf Temperaturen zwischen 130 bis 150 °C erhitzt und danach schnell wieder abgekühlt. Ein anderes Verfahren ist die Pasteurisation mit niedrigeren Temperaturen, ähnlich wie bei frischer Vollmilch. Hierbei wird der Saft einige Sekunden auf ca. 85 °C erhitzt. Ein derart konservierter Saft ist ungekühlt ca. 12-18 Monate haltbar.

Gemäß einer weiteren Ausführungsform enthält das erfindungsgemäße Getränk 200 bis 1000 mg/l EGCG und 80 bis 350 mg/l NR oder 0,5 bis 5 g/l Tryptophan.

Alternativ zu der Getränkeform oder zur Form als Getränke-Basismittel, wie Sachets, Brausetabletten, Pulverform oder Konzentrate, kann das erfindungsgemäße Mittel auch als pharmazeutisches Präparat in Form von anderen Arzneimitteldosierungen zur Verfügung gestellt werden, z.B. in Form von Tabletten, Kapseln, Dragees, etc.. Dabei können diese mit magensaftresistenten Umhüllungen bereitgestellt werden. Vorzugsweise werden diese Arzneimitteldosierungen in Dosiseinheitsform, insbesondere als Tagesdosis (beispielsweise (auch jeweils unabhängig voneinander) 1 mg bis 10 g, vorzugsweise 1 bis 1000 mg, noch bevorzugter 10 bis 500 mg, insbesondere 50 bis 300 mg, EGCG, 1 bis 500 mg, vorzugsweise 10 bis 100 mg, insbesondere 20 bis 80 mg, NR und/oder 0,05 bis 10 g, vorzugsweise 0,1 bis 5 g, insbesondere 0,5 bis 3 g, Tryptophan sowie gegebenenfalls 0,1 mg bis 500 mg, vorzugsweise 0,5 mg bis 200 mg, insbesondere 1 bis 100 mg, Steviosid; nebst weiteren Inhaltsstoffen, wie pharmazeutischen Trägern oder Geschmacksstoffen), vorgesehen. Vorzugsweise enthalten die Tagesdosen 50 bis 200 mg EGCG und 20 bis 80 mg NR und/oder 0,5 bis 3 g Tryptophan sowie gegebenenfalls einen Süßstoff, insbesondere einen Süßstoff, umfassend Steviosid und/oder Rebaudiosid A (vorzugsweise in einer Menge von 1 bis 80 mg). Das erfindungsgemäße pharmazeutische Präparat kann insbesondere auch in Form eines Nahrungsergänzungsmittels zur Verfügung gestellt werden. Besonders bevorzugte Tagesdosierungen enthalten, unabhängig voneinander, 80 bis 150 mg, insbesondere 100 bis 130 mg, EGCG, 30 bis 70 mg, insbesondere 40 bis 60 mg, NR und/oder 1 bis 3 g Tryptophan. Diese Tagesdosen können natürlich, wie oben erwähnt, auch als Getränk zur Verfügung gestellt werden, vorzugsweise in einem Volumen von 100 bis 1000 ml, noch bevorzugter von 200 bis 750 ml, insbesondere 250 bis 500 ml.

Gemäß einem weiteren Aspekt betrifft die vorliegende Erfindung ein Kombinationspräparat, umfassend EGCG und NR (und/oder Tryptophan; wobei erfindungsgemäß die NR-Variante bevorzugt ist) als Wirksubstanzen, insbesondere zur Verwendung als Mittel zur Konzentrationssteigerung. Die erfindungsgemäße Kombination von EGCG und NR hat sich besonders bewährt bei der Steigerung der Konzentration. Die Erfindung betrifft auch die Verwendung eines Kombinationspräparats, umfassend EGCG und NR als Wirksubstanzen, als Mittel zur Konzentrationssteigerung, wobei dieses Kombinationspräparat vorzugsweise als Getränk oder Getränkebasis oder als pharmazeutisches Präparat wie hierin beschrieben vorliegt.

EGCG kann prinzipiell zwar auch in einer Dosis von unter 1 mg eingenommen werden, jedoch muss dann die erfindungsgemäße Wirkung nicht unbedingt eintreten. Bei einer Dosis von über 10 g wird zwar die konzentrationssteigernde Wirkung erzielt, es kann bei derart hohen Dosen aber zu Nebenwirkungen kommen, die unerwünscht sind, wie z.B. Einschlafproblemen oder anderen nervösen Begleiterscheinungen. Prinzipiell sind für die regelmäßige Einnahme eher geringere Dosierungen zu wählen; für die Einmaleinnahme zur Herbeiführung einer zeitnahen Konzentrationssteigerung können eher höhere Dosen sowohl von EGCG als auch von NR (oder Tryptophan) genommen werden. Üblicherweise (auch dies jedoch oft abhängig von der Dosierung) tritt die konzentrationssteigernde Wirkung etwa eine halbe Stunde bis eine Stunde nach der (oralen) Einnahme des erfindungsgemäßen Mittels ein und hält dann, je nach Dosis, über einen längeren Zeitraum, z.B. mehrere Stunden, an. Während dieser Zeit äußert sich die konzentrationssteigernde Wirkung sowohl in der Einzelleistung (d.h. dass das individuelle Konzentrationsvermögen gesteigert wird) als auch in anderen Konzentrationsparametern, insbesondere in einer Verzögerung oder Verhinderung des zeitlichen Konzentrationsabbaus (also in einer Erhöhung der geistigen Ausdauer).

Demgemäß liegen bevorzugte Dosierungen (unabhängig davon, ob das erfindungsgemäße Mittel als Getränk, als Arzneimittel-Dosisform oder in anderer Form eingenommen wird) für eine Einmaldosierung (z.B. zur schnellen Herbeiführung eines gesteigerten und länger andauernden Konzentrationsvermögens) bei 10 mg bis 10 g EGCG und 1 bis 100 mg NR, vorzugsweise 100 mg bis 5 g EGCG und 2 bis 50 mg NR, insbesondere 500 mg bis 4 g EGCG und 5 bis 40 g NR (bzw. bei 10 mg bis 10 g EGCG und 0,1 bis 10 mg Tryptophan, vorzugsweise 100 mg bis 5 g EGCG und 0,5 bis 5 g Tryptophan, insbesondere 500 mg bis 4 g EGCG und 1 bis 4 g Tryptophan). Eine kontinuierliche Einnahme des erfindungsgemäßen Mittels kann z.B. einmal täglich, einmal alle zwei Tage, einmal alle drei Tage erfolgen, jedoch können Einzeldosierungen auch mehrmals täglich (zweimal, dreimal, viermal, fünfmal, etc.) erfolgen. Dabei sollte darauf geachtet werden, dass einerseits die Dosis hoch genug ist, um einen erhöhten Level von EGCG und NR (bzw. Tryptophan) im Körper aufrechtzuerhalten; andererseits sollte man vor allem Überdosierungen von EGCG vermeiden (s. oben).

Die Einnahme des erfindungsgemäßen Kombinationspräparates kann einerseits bereits in kombinierter Form erfolgen (dies ist jedenfalls bei der Einnahme als Getränk auch die bevorzugte Variante); andererseits kann das erfindungsgemäße Kombinationspräparat aber auch in getrennter Form eingenommen werden, z.B. kann die EGCG-Komponente in fester Form (z.B. als Pulver, Kapsel oder Tablette oral) und die NR-Komponente in Form einer wässrigen Lösung oder Suspension (z.B. ebenfalls oral) davor oder danach eingenommen werden. Demgemäß betrifft die vorliegende Erfindung auch ein Kombinationspräparat, umfassend EGCG und NR und/oder Tryptophan als Wirksubstanzen, insbesondere zur Verwendung als Mittel zur Konzentrationssteigerung, wobei das Kombinationspräparat aus mindestens zwei getrennt voneinander einnehmbaren Komponenten besteht und in der einen Komponente EGCG und in der anderen Komponente NR und/oder Tryptophan vorgesehen wird.

Die Erfindung wird anhand der nachfolgenden Beispiele und der Zeichnungsfigur näher erläutert, ohne jedoch darauf eingeschränkt zu sein.

Es zeigen:
Fig. 1 und 2 den funktionellen Zusammenhang zwischen NR, NAD, Tryptophan und Sirtuinen;

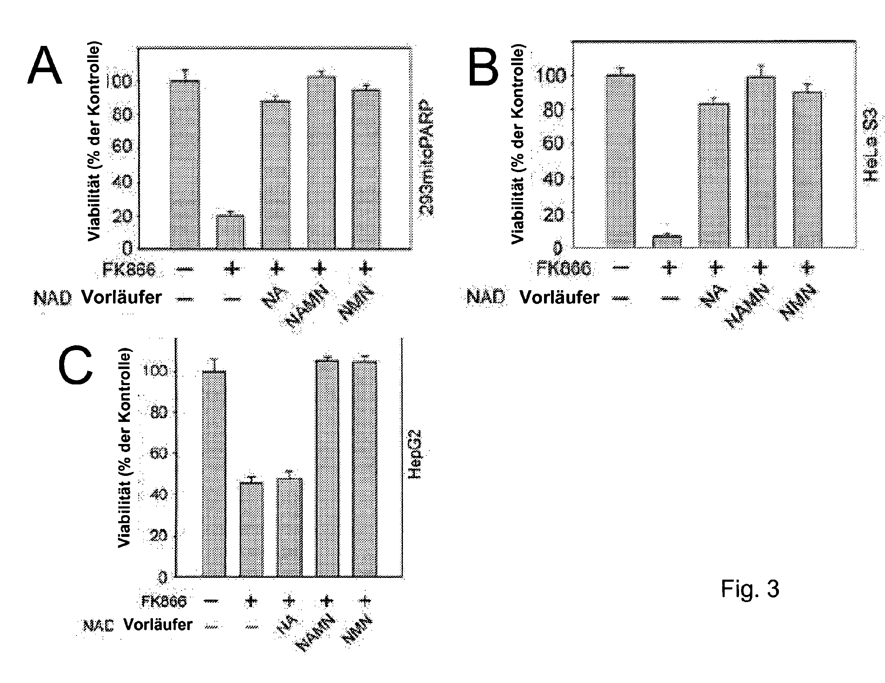

Fig. 3 die Unterstützung der mitochondrialen NAD⁺-Generierung und Überleben der Zellen durch extrazelluläre NAD⁺-Derivate; Nam Nutzung wurde von FK866 gehemmt; sowohl NAMN als auch NMN unterstützen mitochondriale NAD⁺-Bildung und schützen 293mitoPARP- (A) und HeLa S3-Zellen (B) vor FK866-induziertem Zelltod; Mononukleotid-Vorläufer, aber nicht NA, unterstützen die Generierung von mitochondrialem NAD⁺ und Lebensfähigkeit von HepG2-Zellen (C);

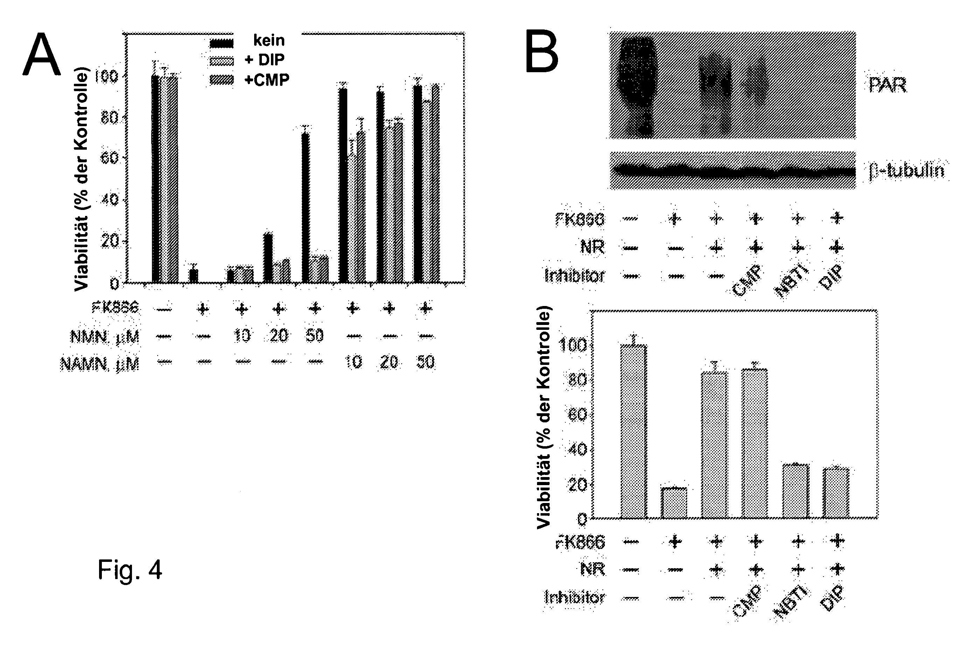

Fig. 4 den Abbau der extrazellulären Nukleotid-Vorstufen und die Aufnahme der resultierenden Riboside in die Zelle; wenn NMN oder NANM als NAD⁺-Vorstufen verwendet werden, hängt die Zellviabilität vom extrazellulären Abbau der Mononukleotide ab (A); extrazelluläres NR tritt in die Zelle über Nukleosid-Transporter ein und unterstützt die mitochondriale NAD⁺-Generierung und die Zellviabilität (B);

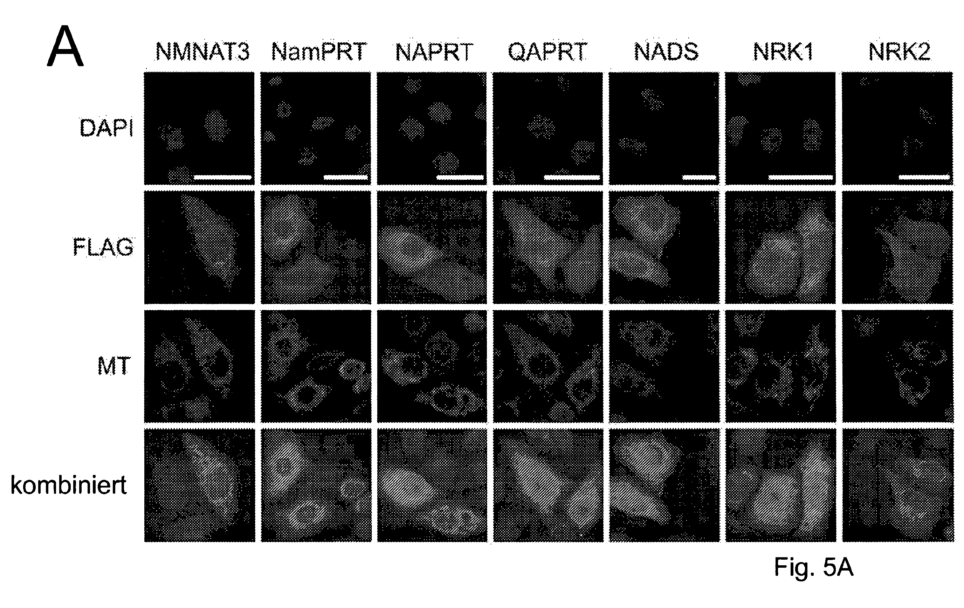

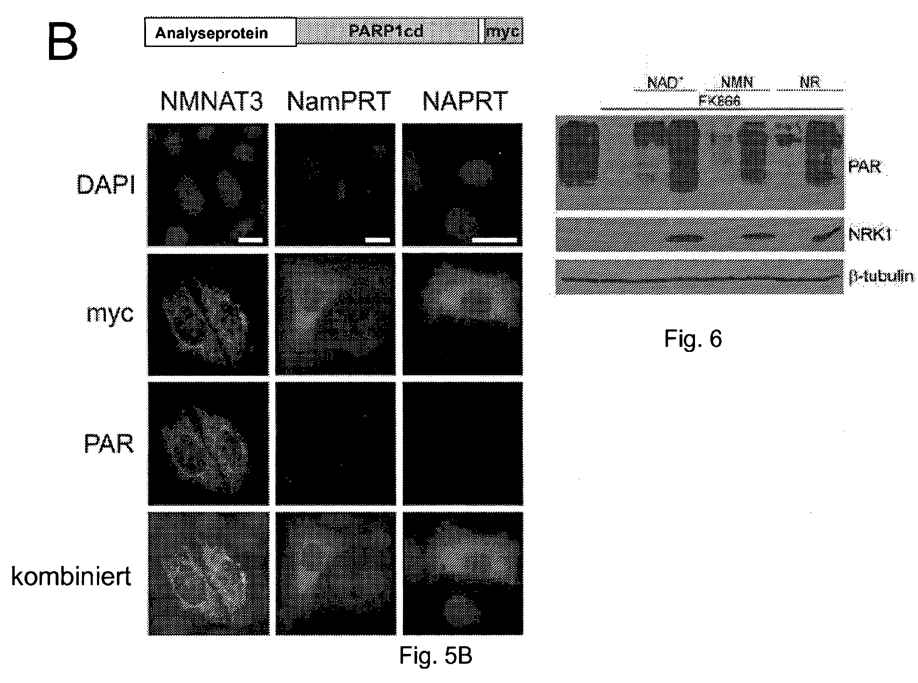

Fig. 5 die subzelluläre Lokalisation der NAD⁺-Biosyntheseenzyme; A: Alle bekannten NAD⁺-Biosyntheseenzyme lokalisieren im Zytoplasma oder Nukleus, mit Ausnahme der mitochondrialen NMNAT3; C-terminal FLAG-markierte Proteine wurden transient in HeLa S3 Zellen exprimiert; die Fluoreszenz-Aufnahmen zeigen Kerne (DAPI), exprimierte rekombinante Proteine (FLAG) und Mitochondrien (MT) (Balken: 20 µm); B: NMNAT3, aber nicht NamPRT oder NAPRT, ist ein mitochondriales Matrixprotein; submitochondriale Proteinlokalisation wurde nach Überexpression in HeLa S3 Zellen mit Hilfe von PARAPLAY durchgeführt; PAR Anhäufung zeigt Matrix-Lokalisation des Myc-Tag Analyt-PARP1cd Fusionsprotein (Balken: 20µm); und
Fig. 6 dass NMN die zytosolische Vorstufe mitochondrialen NAD⁺-Synthese ist; die Erhöhung des mitochondrialen NAD⁺-Gehalts durch Überexpression von NRK1 hängt von einer extrazellulären NR-Quelle ab (NMN, NAD⁺ oder NR, wie angegeben); zytosolisches NMN dient als Vorläufer; erhöhte NRK-Aktivität erhöht den mitochondrialen NAD⁺-Gehalt wenn NR, NMN oder NAD⁺ als extrazelluläre NAD⁺-Vorstufen vorgesehen werden.

### BEISPIELE:

### 1.: Herstellung des erfindungsgemäßen Getränks

In einem Liter karbonisiertem Felsquellwasser werden
- 100 mg EGCG,
- 10 mg NR,
- 1,2 kg kristalline Zitronensäure bzw. die hierzu äquivalente Menge flüssige Zitronensäure, und
- 300 mg Stevia-Extrakt zugegeben und darin aufgelöst. Dabei sind dem Wasser pro Liter etwa 7 g (ca. 6,8 bis ca. 7,2 g) CO₂ zugesetzt.

In einem Liter karbonisiertem Felsquellwasser werden
- 50 mg EGCG,
- 10 mg NR,
- 1 g Tryptophan,
- 1,2 kg kristalline Zitronensäure bzw. die hierzu äquivalente Menge flüssige Zitronensäure, und
- 30 mg Steviosid
zugegeben und darin aufgelöst. Dabei sind dem Wasser pro Liter etwa 7 g (ca. 6,8 bis ca. 7,2 g) CO₂ zugesetzt.

In einem Liter frisch gepresstem Orangensaft werden
- 500 mg EGCG,
- 50 mg NR,
- 2 g Tryptophan, und
- 30 mg Steviosid
zugegeben und darin aufgelöst.

In einem Liter frisch gepresstem Apfelsaft werden
- 500 mg EGCG,
- 80 mg NR,
- 2 g Tryptophan, und
- 50 mg Stevia-Extrakt
zugegeben und darin aufgelöst. Dann wird dieses Apfelsaftgemisch mit einem Liter karbonisiertem Felsquellwasser versetzt.

In einem Liter karbonisiertem Felsquellwasser werden
- 2 g EGCG und
- 20 mg NR,
zugegeben und darin aufgelöst.

In einem Liter karbonisiertem Felsquellwasser werden
- 1 g EGCG und
- 50 mg NR,
zugegeben und darin aufgelöst.

In einem Liter karbonisiertem Felsquellwasser werden
- 2 g EGCG und
- 80 mg NR,
   zugegeben und darin aufgelöst.

In einem Liter karbonisiertem Felsquellwasser werden
- 2 g EGCG,
- 50 mg NR,
- 1,0 kg kristalline Zitronensäure bzw. die hierzu äquivalente Menge flüssige Zitronensäure, und
- 30 mg Steviosid
zugegeben und darin aufgelöst.

In einem Liter karbonisiertem Felsquellwasser werden
- 1 g EGCG,
- 1 g Grünteeextrakt (standardisiert auf 50 % EGCG),
- 100 mg NR,
- 1,0 kg kristalline Zitronensäure bzw. die hierzu äquivalente Menge flüssige Zitronensäure, und
- 30 mg Steviosid
zugegeben und darin aufgelöst.

### 2.: Konzentrationssteigernde Wirkung des erfindungsgemäßen Getränks im Vergleich mit EGCG alleine

### 2.1.: d2-Aufmerksamkeits-Belastungstest und FAIR (Frankfurter Aufmerksamkeits-Inventar)

Die konzentrationssteigernde Wirkung des erfindungsgemäßen Getränks kann speziell im Vergleich mit bekannten Mitteln zur Konzentrationssteigerung in Standardtests zur Bestimmung der Konzentration bestimmt werden. Dabei wird vor allem die verstärkende Wirkung von Tryptophan und insbesondere NR nachweisbar, vor allem in den besonderen Vergleichsgruppen: Schüler im Alter von 10 bis 18 Jahren, berufstätige Frauen und Männer von 30 bis 40 Jahren, von 35 bis 45 Jahren und von 40 bis 50 Jahren, sowie Pensionisten von 60 bis 70 Jahren und von 65 bis 75 Jahren.

Die dabei verwendeten Tests sind vorzugsweise der d2-Aufmerksamkeits-Belastungstest und FAIR (Frankfurter Aufmerksamkeits-Inventar) (weitere: z.B. in Westhoff/Hagemeister, "Konzentrationsdiagnostik" (2005), Seiten 41 - 55).

Dabei lassen sich auch für die jeweiligen Personengruppen die Untergruppen an wirksamen Dosierungen für EGCG, NR und/oder Tryptophan ermitteln, bei denen gerade noch ein statistisch signifikanter Unterschied zwischen EGCG alleine und dem erfindungsgemäßen Getränk in den jeweiligen Gruppen feststellbar ist.

### 2.2. Tripel-Test Verfahren zur Bestimmung der Aufmerksamkeit

### 2.2.1. Zusammenfassung des experimentellen Set-Ups:

Im Folgenden wird die vorliegende Erfindung im Rahmen der viewpointsystem^{®}-Blickanalysen, unter Verwendung von standardisierten Untersuchungsmethoden des Wiener Testsystems und mit biomedizinischen Untersuchungen zur Stressforschung, getestet. Dabei wird im Blicklabor das Tripel-Test Verfahren mit höchster Genauigkeit zur Bewertung der psychophysischen Einflüsse bis hin zum Aufmerksamkeitsgrad eingesetzt (vgl. EP 1 300 108 A1, WO 2008/151346 A1).

Die hohe Aussagekraft des Tripel-Test Verfahrens von view-pointsystem^{®} besteht darin, dass die Kombination der international wissenschaftlich abgesicherten Messverfahren wie die neue viewpointsystem^{®}-Blickforschung, das psychologische Wiener Testsystem und die Biomedizinische Testbatterie die exakte nachvollziehbare Beurteilung der Auswirkungen von konzentrationsfördernden (leistungsstärkenden) Substanzen garantiert.

12 Probanden wurden in 3 Gruppen von jeweils 4 Probanden unterteilt:
(1) erfindungsgemäßes Getränk (150 mg EGCG/20 mg NR in 10 ml H₂O; Gruppe "Energy" bzw. "Energy-Drink")
(2) EGCG alleine (150 mg EGCG; Gruppe "Grüntee")
(3) Plazebo (H₂O und Mannitol; Gruppe "Plazebo")

### 2.2.2. Das Tripel-Test-Untersuchungsverfahren:

Die Probanden wurden dann mit Hilfe des Tripel-Test-Untersuchungsverfahrens, das sind vps-Blickanalysen - Wiener Testsystem - Biomedizinische Untersuchungsbatterie, in einer standardisierten Untersuchung systematisch hinsichtlich allfälliger Einflüsse untersucht, sodass objektive, statistisch signifikante Aussagen zum Nachweis der konzentrationssteigernden Wirkung des erfindungsgemäßen Getränks möglich wurden.

Mit diesem Testsystem wurden die Änderungen in der Aufmerksamkeit, dem Blickverhalten, der Reaktion, der Aktivation und der Stressfaktor bei den drei Gruppen ermittelt. Hierbei wurden die realen Blickrichtungen und Blickbewegungen von Probanden im Zuge des Wiener Testsystems - beim Reaktions- und Determinationstest, sowie beim Betrachten von bestimmten Situationsabläufen (normiert vorgegebene Bild- und Filmsequenzen mit Ereignissen) und bei ausgewählten spezifischen Eignungstests - aus dem jeweiligen Gesichtsfeld gefilmt und eingespielt (vgl. EP 1 300 108 A1, WO 2008/151346 A1).

Durch die zeitliche und örtliche Dokumentation der Blickzuwendungen konnten die Blickpunkte in der Bewegung eindeutig dokumentiert werden, wobei insbesondere die Blickeinsätze bzw. Blickhäufigkeiten für jede Situation genau erfasst, normiert und offengelegt wurden. Durch dieses Verfahren war es daher möglich die Unterschiede bei den Blickbewegungen herauszuarbeiten, psychophysische Reaktionszeiten und Blickbindungen zu analysieren und sehphysiologische Blicklatenzen genau zu dokumentieren. Darüber hinaus konnten über den Lidschlag die Belastungsgrößen verifiziert werden. Damit kann der Blick nicht nur nach Ort und Zeit analysiert (wo schaut man wie lange hin), sondern auch die Wahrnehmungsqualität beurteilt werden. Mit den viewpoint-High-Tech-Programmen wird das Blickverhalten von Personen mit einer Genauigkeit von 15 Bogenminuten bzw. 40-Tausendstel Sekunden analysiert. Diese Blickverhaltensforschung klärt daher exakt alle Formen von Blickabsenzen, Informationsdefekten, Informationsdefiziten, Blickbindungen und Überinformationen (komplexe Situationen als Hauptgründe für Fehlhandlungen u.a.) auf. Ausgewiesen werden auch:
- Das Scharfsehen (foveale Genauigkeit)
- Die Unterscheidung von Fixationen (Blickpunkten) und Sakkaden (Blicksprüngen)
- Die Messung und Bewertung der Komplexität (hohe Anforderungen) und Informationsdichte (Informationen in der Zeiteinheit).

Parallel dazu wurden auch die relevanten biomedizinischen Kennwerte wie Hautleitwert, Puls, miterfasst und ausgewertet. Insgesamt wurden daher im Zuge der Tripel-Test-Untersuchungen für alle handlungsrelevanten Beurteilungssituationen Blickuntersuchungen im Blicklabor durchgeführt, das Blickverhalten und die Reaktionszeiten analysiert. Die Auswirkungen auf die Informationsaufnahme, einschließlich Informationsstress und Müdigkeit, wurden mit den standardisierten Blickverfahren durch Raster-Analyse-Programme ausgewiesen.

Durch die Dokumentation der Blickzuwendungen bei den vorgeführten Sequenzen konnten physiologische Leistungsgrenzen, Blickabsenzen, störende Blickbindungen und sonstige menschliche Fehlerhäufigkeiten genau offengelegt und erfasst werden. Die viewpointsystem®-Blickforschung kombiniert mit der Erfassung der biomedizinischen Kenndaten lässt die Verhaltens- und Reaktions-änderungen von leistungsbeeinflussten und unbeeinflussten Probanden genau unterscheiden und ermöglicht somit eine objektivierte Erfassung von Konzentrationsabläufen.

### 2.2.3.: Auswerteverfahren (s. auch: EP 1 300 108 A1, WO 2008/151346 A1):

Als Ergebnis wurde das Blickverhalten auf die Detaildarstellungen hinsichtlich Blickreihenfolge, Blickhäufigkeiten nach Kategorien, Angaben von Blickbindungen u.a. aufgezeichnet, sodass insgesamt eine objektive Bewertung möglich war. Die Feststellung der Komplexität, Vielfältigkeit und Detailerkennung erfolgte durch FPC - FOVEAL PERCEPTION CIRCLES-Blickfilme, die Bestimmung der Blickhäufigkeiten und Blickdauern (Offenlegung der Zeitökonomie) wurde durch dynamische Punktwolkengrafiken in den TIME BUBBLES (TB) -Blickfilmen offengelegt. Es wurden Detailanalysen mit folgenden Rasteranalyseprogrammen vorgesehen, wobei die Gesamtergebnisse je nach Inhalt in geeigneter Form visualisiert werden:
Einsatz von Rasteranalyseprogrammen:

Das viewpointdynamicANALYsis Analyse-Modul (gemäß EP 1 300 108 A1, WO 2008/151346 A1) ist das derzeit modernste Analyseverfahren zur Findung und Bestimmung von Blickabsenzen und Blickdefekten (Störungen der geordneten Informationsaufnahme) mit der gleichzeitig ablaufenden dynamischen Darstellung von vorzugsweise FPC-Blick-Videos und den zu gehörigen viewpoint-Sequenzen. Mit Hilfe dieser Detailanalysen wurden die Bereiche, die hohes Hauptinteresse, hohe Detailerkennungswerte und beste Zeitökonomie darstellen, jenen Bereichen gegenübergestellt, wo offensichtlich sehphysiologisch wenig Interesse, geringe Detailerkennung und schlechte Zeitdispositionen auftraten bzw. echte Informationsfehler und Informationsdefekte nachweisbar wurden.

Die laufende visuelle Darstellung der Fixationsdominanzen und Sakkadendominanzen erfolgte mit dem viewpointcomplexityFIN-DER auf Grundlage des priorityzone-Algorithmus, sodass die Bereiche der hohen Komplexität direkt in einer Doppel-Filmdarstellung eingesehen werden konnten. Durch dieses High-Tech-Detektionsprogramm konnten die laufende Beurteilung der Fixationsleistung und Sakkadenbewegungen (Informationsausfälle, Informationsdefekte) bereits im Film erfolgen. Gleichzeitig wurde die Informationsdichte (Grad der Komplexität) des jeweiligen Ausschnittes zahlenmäßig als Wert ausgewiesen und besonders anschaulich über einen seitlichen Farbbalken visualisiert.

Die Analysen mit dem viewpointcomplexityRESEARCH - Analyseprogramm dienten zur Findung und detaillierten Untersuchung von Stellen mit Informationsverlusten, bedingt durch hohe Komplexität oder häufige foveale zentrale Blickbindungen. Mit diesen Parametern waren daher qualitative Tiefenanalysen der realen Informationsaufnahme und verfeinerte Betrachtungen von unterschiedlichen Informationsausfällen und Informationsdefekten möglich.

Detaillierte Analysen des Lidschlages zur Bestimmung von Informationsstress, des Belastungsgrades und der Müdigkeit erfolgten mit dem viewpointeyeblinkRESEARCH - Programm. Es konnte abschnittsweise die Lidschlagrate, die Lidschlagdauer, unter besonderer Berücksichtigung von flurries bzw. burst of blinks zur Feststellung der Aufmerksamkeitsrate bzw. Monotonie bzw. des Übermüdungsfaktors ausgewiesen werden.

Die laufende Zusammenschau und Beurteilung der räumlichen Häufigkeitsverteilungen im Sichtfeld erfolgte nach 50%, 85%, 95% Verteilungen mit Erkennung und Ausweisung des Schwerpunkt-Bereiches. In Detailanalysen wurden die Bereiche, die hohes Hauptinteresse, hohe Detailerkennungswerte und beste Zeitökonomie darstellen, jenen Bereichen gegenübergestellt, wo offensichtlich sehphysiologisch und emotional wenig Ansprache, geringe Detailerkennung und schlechte Zeitdispositionen auftreten.

### Detailauswertung physiologischer Parameter:

Die Analyse der stressverursachenden Faktoren und Abläufe in realen Situationen ist mit dem mobilen Kleinstaufnahmegerät Physio-Recorder möglich. Dadurch können die Aktivierungs- und Stresszustände von Personen aufgezeigt werden.

Der Proband trägt nur eine kleine Einheit - etwa in der Größe einer Armbanduhr - sowie die Elektroden direkt an der Hand bzw. am Ohr. Auch hier ist bei uneingeschränkter Bewegungsfreiheit eine umfassende Aufzeichnung der Körperreaktionen mit höchster Genauigkeit möglich.

Nach einer Baseline-Messung folgt die Untersuchung, wobei u.a. folgende Werte aufgezeichnet werden:
- Herzfrequenz (HF: Anzahl der Herzschläge/Minute)
- Blutvolumenpuls (BVP: Maß für Blutmenge welche pro Herzschlag durch den Körper gepumpt wird)
- Hautleitwert (SCL: Skin Conductance Level/Leitwertsniveau, Tonisches Maß, SCR: Skin Conductance Response/Phasisches Maß; Reaktion auf einzelne Reize)

Dadurch ist es möglich, Aktivierungsvorgänge, Anpassungsleistungen und Stresssituationen eindeutig zu erkennen. Durch den gemeinsamen synchronen Einsatz dieses Systems und der Blickerfassung wird der Zusammenhang zwischen der Informationsaufnahme und der körperlichen Reaktion erstmals offengelegt, wodurch wesentlich detailliertere Aussagen über die Ursachen von Belastungen und Stress- bzw. Aktivierungszuständen getroffen werden.

### 2.2.4.: Ergebnisse:

### 2.2.4.1.: Zusammenfassung der Ergebnisse der Untersuchung "ENERGY-Drink" aus den Detailanalysen der physiologischen Parameter

Bei den Detailanalysen wurden synchron die blicktechnischen und biomedizinischen Daten der Untersuchungssequenzen [Reaktionstest (singuläre Reize), Determinationstest (multiple optische, akustische Reize), Filmbetrachtung (Realsequenzen aus Lenkersicht und Filmtrailer) und Semiotik (Tierabbildungen und Logos)] ausgewertet. Besonderes Augenmerk wurde auf die Hautleitwert-Level (SCL), die Herzrate (Puls; HR) und die Atemfrequenz (AF) gelegt.

Diese Parameter sind Hinweise auf sympathische Aktivierungen der Probanden. Der Sympathikus ist neben dem Parasympathikus und dem enterischen Nervensystem ein Teil des vegetativen Nervensystems. Die meisten Organe werden von den ersten beiden Systemen gesteuert, die als Gegenspieler (antagonistisch) wirken und dadurch eine äußerst feine Regulation der Organtätigkeit ermöglichen. Der Sympathikus hat in diesem System eine ergotrope Wirkung, das heißt, er erhöht die nach außen gerichtete Handlungsbereitschaft.

Der Sympathikus aktiviert bei Einwirkung von Stressreizen alle Notfallfunktionen des Organismus, die diesen in eine erhöhte Handlungsbereitschaft versetzen: Puls und Blutdruck steigen an, der Blutglukosespiegel steigt, um eine rasch verfügbare Energiequelle zu erschließen, der Aufmerksamkeitslevel wird gesteigert. Ist die Situation vorüber, gewinnt der Parasympathikus das Übergewicht: Puls und Blutdruck verlangsamen sich, die im Blut zirkulierende Glukose sinkt wieder ab. Der Organismus ist auf Ruhe geschaltet, um Erholung für zukünftige Ereignisse zu gewährleisten.

Nach Ermittlung der physiologischen Parameter wurden für jeden Abschnitt die statistischen Parameter Mittelwert/Standardabweichung/Min/Max/Spannweite/85%-Perzentil ermittelt. Weiters wurden die Trendgeraden-Gleichungen berechnet.

Zur weiteren statistischen Detailauswertung wurden die 85%-Perzentilwerte herangezogen und die relativen Unterschiede mittels der Verhältniszahl nachher/vorher erhoben. Diese Änderung wurde als Maßzahl summiert.

Nach Kategorisierung der Probanden in 3 Vergleichsgruppen wurden die Maßzahlen aufsummiert und stellen ein Maß für die physiologische/mentale Belastung der Probanden dar. Je niedriger die Zahl umso "gelassener" werden die Aufgabenstellungen von den Testpersonen gelöst.

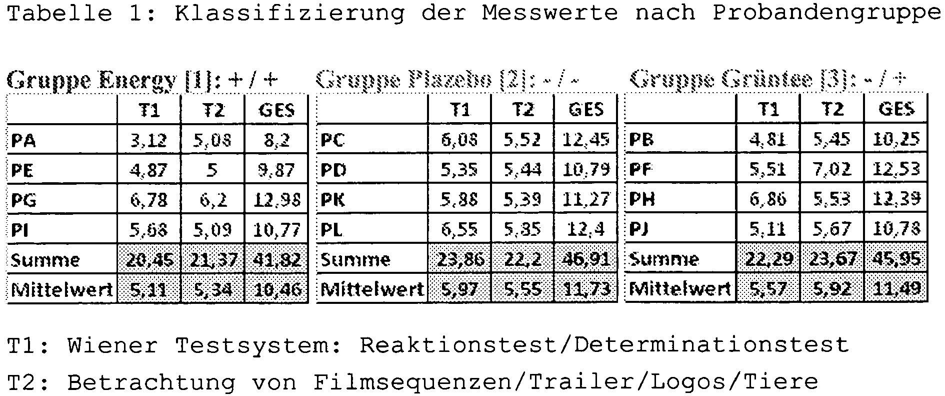

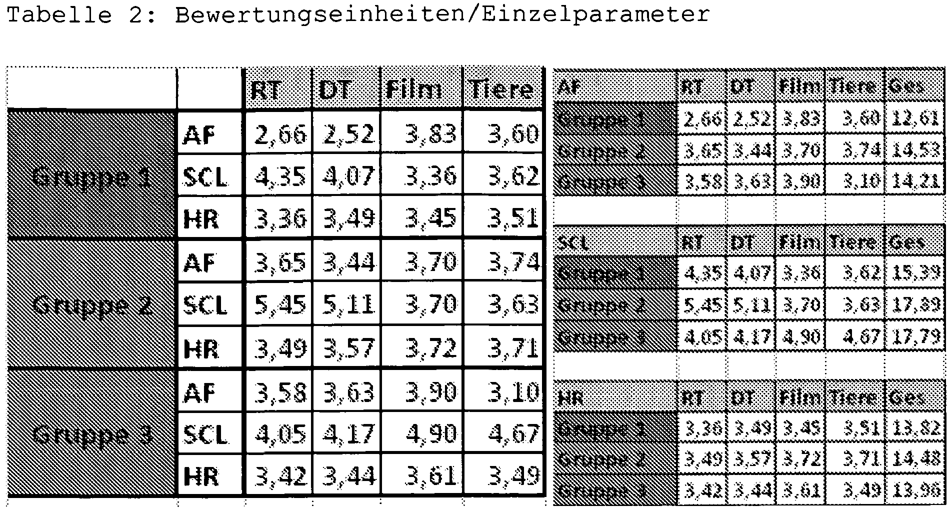

Die Auswertung der Summenparameter zeigt tendenziell vor allem bei den aktiven Testsequenzen (Manipulationen der Probanden erforderlich) eine höhere Leistungsfähigkeit für die Problemlösungen (höhere Belastbarkeit) der Probanden der Gruppen "Energy" und "Grüntee". Die Probandengruppe "Energy" ist nach Auswertung der biomedizinischen Parameter Atemfrequenz, Hautleitwert und Herzrate geringer gestresst.

### 2.2.4.2.: Beschreibung der Detailanalysen des Wiener Testsystems:

### Untersuchungen

### Reaktionstest (RT)

Nach Dorsch ("Psychologisches Wörterbuch", (12. Auflage) (1994), Hrsg.: Häcker et al., Huber Verlag, Bern) ist "Reaktionszeit" die Zeit, die zwischen einem Signal und dem Beginn der mechanischen Bewegungsantwort vergeht, unter der Instruktion, möglichst schnell zu reagieren. Da es sich hier um Genauigkeiten im Millisekundenbereich handelt, muss das eingesetzte Testinstrument sehr zuverlässig und genau sein. Der Reaktionstest erlaubt durch spezielle Testformen die messgenaue Erfassung der Reaktionszeit und Motorischen Zeit.

### Durchführung:

Als Eingabemedium dient die Probandentastatur. Eine animierte Instruktionsphase und eine fehlersensitive Übungsphase führen in die Aufgabenstellung ein. Im Rahmen der Testvorgabe werden den Probanden Farbreize und akustische Signale vorgegeben. Der Proband erhält die Instruktion, die Reaktionstaste nur bei Darbietung relevanter Reize zu drücken und anschließend den Finger sofort wieder auf die Ruhetaste zurückzulegen.

### Determinationstest (DT)

Der Determinationstest ist ein besonders messgenaues Verfahren zur Erfassung der reaktiven Belastbarkeit und dient der Erfassung der reaktiven Belastbarkeit sowie der damit verbundenen Reaktionsfähigkeit. Das Verfahren erfordert als kognitive Teilleistungen die Unterscheidung verschiedener Farben und Töne, das begriffliche Fixieren der relevanten Merkmale von Reizkonfiguration und Bedienungselementen, sowie der Zuordnungsregeln und das Auswählen der relevanten Reaktion nach den per Instruktion vereinbarten und/oder im Testverlauf erlernten Zuordnungsregeln. Das Belastende beim DT liegt im fortlaufenden, möglichst anhaltend schnellen und unterschiedlichen Reagieren auf schnell wechselnde Reize.

Die Anwendung liegt in der Messung der reaktiven Belastbarkeit, der Aufmerksamkeit und der Reaktionsgeschwindigkeit bei fortlaufend geforderten schnellen und unterschiedlichen Reaktionen auf rasch wechselnde optische und akustische Reize.

### Durchführung

Dem Probanden werden Farbreize und akustische Signale vorgegeben. Die Reaktion erfolgt durch Betätigung der korrespondierenden Tasten auf der Probandentastatur. Die Reizdarbietung erfolgt adaptiv, die Vorgabegeschwindigkeit wird an das Leistungsniveau des Probanden anpasst.

Auf Basis dieser Ergebnisse wurden nunmehr die Blickuntersuchungen durchgeführt. Neben den genauen Messungen von Blicklatenzen (verspätete Zuwendungen), Adaptationsparameter wie Änderungsgeschwindigkeit und Dauer wurden zur Definition von Stressgrößen auch die Lidschlagdauern und Lidschlaganzahl ausgewiesen.

### Ergebnisse aus den synchronen Blickuntersuchungen

Die Blicklatenzen bei realen Filmsequenzen (die verspäteten Blickzuwendungen) sind bei der Probandengruppe Energy für die Auswertung von realen Filmsequenzen (Realsequenz) am geringsten - es zeigen sich Bestwerte für diese Gruppe im jeweiligen Nachher-Zustand. Während vor allem die Plazebogruppe extreme Steigerungsraten und die Grünteegruppe geringe Steigerungen aufweist, weist die Energygruppe keine großen Steigerungen auf.

Die Gesamtauswertung für Lidschlagdauern und Lidschlaganzahl für alle Testverfahren (wie Reaktionstest, Determinationstest und Realsequenzen) zeigt für die Probandengruppe Energy bis zu 21 % kürzere Lidschlagdauern als für die Plazebogruppe und Grünteegruppe. Geht man davon aus, dass die Lidschlagdauer als Maß für besseres zeitökonomisches Handling bei diversen Tätigkeiten zu definieren ist und gleichzeitig die Anzahl der Lidschläge bei der Placebogruppe und der Grünteegruppe deutlich geringer ist, so kann für diese beiden Gruppen ein höherer Stresszustand erkannt und umgekehrt für die Energygruppe eine verbesserte Bewältigung bei Problemlösungen interpretiert werden. Dieses Ergebnis deckt sich mit den biomedizinischen Ergebnissen aus dem Wiener Testsystem.

Bei der Auswertung von Pupillengrößen und den Änderungsgeschwindigkeiten wurden im Detail die Änderungen bei Filmszenen von dunkel auf hell und von hell auf dunkel untersucht, die Pupillengrößen gegenübergestellt und die Änderungsgeschwindigkeiten ausgewiesen. Hierbei zeigen sich folgende Zusammenhänge: Vor allem bei Änderungen von dunkel auf hell sind für die Energygruppe deutlich steilere Änderungsmaße gegeben, was bedeutet dass in dieser Gruppe offenbar das Auge bei den Änderungen dunkel auf hell etwas schneller reagiert, als bei der Plazebogruppe. Diese Aussagen über die Adaptationsneigung lassen auch auf eine Verbesserung der Aktivation schließen.

Insgesamt belegen diese Ergebnisse, dass sich mit dem erfindungsgemäßen Mittel eine gegenüber den Vergleichsgruppen deutliche Konzentrationssteigerung erzielen lassen kann. Insbesondere schlug sich dies in einer Verbesserung der der geistigen Ausdauer in der Energygruppe nieder.

### 3.: Biochemische Charakterisierung

Die Wirksamkeit der erfindungsgemäßen Zusammensetzung kann auch biochemisch nachgewiesen werden, z.B. durch die in Dölle et al. (Anal. Biochem. (385) (2009): 377 - 379) und Berger et al. (J. Biol. Chem. 43 (280) (2005): 36334 - 36341) beschriebenen Methoden.

### 3.1.: Untersuchungen zur subzellulären Kompartimentierung der NAD-Biosynthese in menschlichen Zellen:

### 3.1.1.: Allgemeines:

Die Konzentration von NAD in Zellen und Geweben ist von entscheidender Bedeutung für die Aufrechterhaltung vitaler Funktionen. Zum einen ist NAD ein universeller Energieträger und damit essentiell für die Bereitstellung von ATP, dem Molekül, welches die Mehrzahl aller energieverbrauchenden Prozesse versorgt. Zum anderen ist NAD ein zelluläres Signalmolekül, welches durch spezifische Umwandlungen an der Regulation lebenswichtiger Ereignisse beteiligt ist. Dazu zählen u.a. die Regulation der Genexpression, der Zellteilung, der DNA-Reparatur sowie der Lebensspanne und des Langzeitgedächtnisses. Es ist bekannt, dass die Erhöhung der zellulären NAD Konzentration diese Prozesse positiv beeinflusst.

In menschlichen Zellen wird NAD aus Vitamin B3 synthetisiert. Vitamin B3 umfasst zwei sehr ähnliche Substanzen, Nikotinamid und Nikotinsäure (Nikotinat). Nikotinamid ist unter normalen Bedingungen ausreichend in der Nahrung vorhanden, so dass eine weitere Erhöhung (z.B. als Nahrungsergänzung) nahezu wirkungslos ist. Da Nikotinat über einen alternativen Syntheseweg zum NAD umgewandelt wird, würde eine erhöhte Zufuhr eine aussichtsreiche Strategie zur Erhöhung des NAD darstellen. In der Tat wird Nikotinat therapeutisch als lipid- (bzw. cholesterol-) senkendes Mittel angewendet, wobei diese Wirkungen durch rezeptorvermittelte Mechanismen erklärt werden. Diese Mechanismen scheinen auch die Ursachen für die erheblichen Nebenwirkungen zu sein, unter denen Hautreizungen, Ausschlag und Schwindelgefühl besonders häufig auftreten. Daher ist das Nikotinat ungeeignet, um als Nahrungsergänzung für die Erhöhung des NAD eingesetzt zu werden.

In der neueren Literatur wurde Nikotinamid-Ribosid (NR) als physiologisches Intermediat des NAD-Stoffwechsels beschrieben, welches auf einem alternativen Weg zum NAD umgesetzt wird.

Im Folgenden werden experimentelle Befunde vorgelegt, aus denen hervorgeht, dass NR tatsächlich von menschlichen Zellen aufgenommen und zu NAD umgesetzt werden kann. Obwohl zum Nahrungsgehalt des NR noch wenig bekannt ist, gibt es deutliche Hinweise, dass das NR dort nur in sehr geringen Mengen vorhanden ist. Mit den hierin dargestellten experimentellen Befunden wird aber nachgewiesen, dass mit NR die NAD Konzentration im Gewebe erhöht werden kann, ohne dabei die Nebenwirkungen des Nikotinats hervorzurufen. Weiters konnte beobachtet werden, dass die Zugabe von EGCG zu menschlichen Zellen eine Erhöhung der Expression der NMNAT1, einer Isoform des Enzyms NMNAT (Nikotinamid-Mononukleotid-Adenylyl-Transferase), die in allen Zellen gefunden wird, um 40% bewirken kann. Daraus ergibt sich auch eine physiologisch-wissenschaftliche Erklärung für die konzentrationssteigernde Wirkung des erfindungsgemäßen Getränkes, wobei offensichtlich die kombinierte Gabe von NR und EGCG die zelluläre NAD Synthese durch einen synergistischen Effekt positiv beeinflusst. Einerseits wird NR, zusätzlich zum Nikotinamid in der Nahrung, der NAD Synthese als alternative Vorläufersubstanz zugeführt. Andererseits wird durch EGCG eine notwendige Erhöhung der Kapazität des Enzyms herbeigeführt, welches an beiden Synthesewegen beteiligt ist.

### 3.1.2.: experimentelle Verfahren:

Klonieren und Erzeugung von eukaryontischen Expressionsvektoren Für die Expression der getaggten Proteine, wurden die entsprechenden offenen Leserahmen in PFLAG-CMV-5a (Sigma), pcDNA3.1 (+)-PARP1cd (Dölle et al., Cell. Mol. Life Sci. 67 (2010), 433-443) oder pCMV/myc/mito (Invitrogen) eingefügt. Alle klonierten DNA-Sequenzen wurden durch DNA-Sequenzanalyse verifiziert.

### Pharmakologische Behandlungen

Inhibitoren (2 µM FK866, 2 mM 3-AB, 10 µM NBTI, 2 µM Dipyridamol, 2 mM CMP, 25 µM PPADS, 1 mM Ap4A) oder Metaboliten (100 µM NAD+, 100 µM NAAD, 100 µM NMN, 100 µM NAMN, 100 µM NA, 100 µM NR) wurden dem Zellkulturmedium zugesetzt, wie angegeben. NR wurde wie beschrieben bereitgestellt (Dölle et al., Anal. Biochem. 385 (2009), 377-379). Die Behandlung mit Inhibitoren und Metaboliten wurde für 24-48 h vor der Analyse von PAR Bildung und die Lebensfähigkeit der Zellen durchgeführt (72 h für 293mitoPARP Zellen, 96 h für HeLa S3-Zellen und 120 h für HepG2-Zellen). Die Lebensfähigkeit der Zellen wurde mittels MTT-Assay bestimmt.

### Immuncytochemie

Die Zellen wurden mit 4% (v/v) Formaldehyd in PBS fixiert und mit 0,5% (v/v) Triton X-100 in PBS permeabilisiert. Zellkerne wurden mit DAPI und Mitochondrien mit MitoTracker Red CMXRos (Invitrogen) gefärbt. Bilder wurden mit einem Leica DMI6000B Epifluoreszenz-Mikroskop (Leica Microsystems), ausgestattet mit x10, x40 und x100 Objektiven, aufgenommen.

### Nachweis von Veränderungen im NAD⁺-Gehalt durch die PARP-Aktivität in Mitochondrien

Es wurden 293mitoPARP Zellen bereitgestellt, die ein Fusionsprotein exprimieren ("mitoPARP"), bestehend aus EGFP und PARP1cd (poly-ADP-Ribose-Polymerase-1), welches auf die mitochondriale Matrix abgezielt wurde. Mit mitochondrialem NAD⁺ als Substrat generieren diese Zellen konstitutiv Protein-gebundenes PAR (Protein-gebundene poly-ADP-Ribose), welches immunchemisch visualisiert wird. Variationen im Umfang der erfassten PAR spiegeln damit Veränderungen des mitochondrialen NAD⁺-Gehaltes wider. Ebenso wurde die transiente Expression von mitoPARP in HepG2 und HeLa S3 Zellen verwendet.

### Identifizierung mitochondrialer Matrix-Proteine durch PARAPLAY

Der Poly-ADP-Ribose Assisted Protein Localisation Assay (PARAPLAY) liefert eine luminale Proteinlokalisation (Dölle et al., 2010). Hier wurde PARAPLAY zur Identifizierung mitochondrialer Matrixproteine verwendet. Die zu analysierenden Proteine (NMNAT3, NamPRT und NAPRT) wurden als N-terminale Fusionsproteine mit PARP1cd in HeLa S3 Zellen exprimiert. Aufgrund der niedrigen [NAD⁺] und/oder der hohen PAR-abbauenden Aktivität im Zytosol und im mitochondrialen Intermembranraum konnte kein PAR detektiert werden, wenn das Fusionsprotein in diesen Bereichen vorhanden ist. Im Gegensatz dazu ist Matrix-Lokalisierung leicht durch PAR-Akkumulation erkennbar. Auch wenn die Mehrheit des Fusionsproteins im Zytosol vorhanden ist, reicht ein luminaler Anteil des Proteins aus, um ausreichend immunologisch detektierbares PAR zu erzeugen. Wenn kein PAR detektiert werden kann, wird die Lokalisation des Proteins außerhalb der Matrix durch die Prüfung der Funktionalität des PARP1cd-Teils des Fusionsproteins verifiziert. Dies wird durch Zugabe einer N-terminalen mitochondrialen Targeting-Sequenz zum Fusionsprotein erreicht, womit es in die Matrix geleitet wird und zur PAR-Akkumulation führt.

### 3.1.3.: Resultate:

### In situ Nachweis von relativen mitochondrialen NAD⁺-Spiegeln durch Poly-ADP-Ribose-Generierung in der Matrix

Eine gezielte Expression von PARP1cd in der mitochondrialen Matrix, hier an EGFP fusioniert und als "mitoPARP" bezeichnet, führt zu einer konstitutiven Anwesenheit von PAR innerhalb dieser Organellen. Ein PAR-Signal hängt sowohl von der katalytischen Aktivität von mitoPARP als auch von der Anwesenheit von NAD⁺ ab. Folglich erlaubt die mitoPARP-Expression die Erkennung von Änderungen im mitochondrialen NAD⁺-Gehalt. PAR war in keinem der Experimente im Zellkern nachweisbar, der Ort, an dem endogenes PARP-1 lokalisiert ist.

### Identifizierung von extrazellulären NAD⁺-Metaboliten, welche die mitochondriale NAD⁺-Generierung unterstützen

Es wurde dann untersucht, welche extrazellulären NAD⁺-Vorstufen die mitochondriale NAD⁺-Generierung unterstützen. Jedenfalls erfüllt Nam diese Funktion, weil es in der Regel die einzige NAD⁺-Vorstufe in Zellkulturmedien ist. Wenn daher NamPRT gehemmt wird, muss eine alternative Vorstufe für die NAD⁺-Generierung vorhanden sein. Zugabe von Na, NAMN oder NMN zum Medium konnte den mitochondrialen NAD⁺-Gehalt und das Zell-Überleben wiederherstellen (Fig. 3A). Die Lebensfähigkeit der Zellen korreliert mit dem mitochondrialen NAD⁺-Spiegel, gemäß PAR-Messung. Transfizierte HeLa S3 Zellen zeigten eine ähnliche Empfindlichkeit gegenüber FK866 und Restaurierung des Überlebens durch NA, NAMN und NMN (Fig. 3B). Im Gegensatz dazu konnte NA in HepG2-Zellen keine Unterstützung für die Erzeugung von NAD⁺ und das Überleben der Zelle liefern (Fig. 3C) womit der Mangel an NA-Phosphoribosyltransferase-(NAPRT)-Aktivität in dieser Zelllinie bestätigt wurde.

### Extrazelluläre Nukleotide werden zu den entsprechenden Nukleosiden abgebaut, die dann in die Zellen als NAD⁺-Vorstufen eintreten

Der NPP-Inhibitor PPADS verhindert die Wiederherstellung des mitochondrialen NAD⁺-Pools und das Überleben der Zelle, wenn NAD⁺ als extrazellulären Vorstufe verwendet wurde. Ebenso führte die Zugabe von Diadenosintetraphosphat (Ap4A) als kompetitives NPP-Substrat zu einem ähnlichen Effekt auf die Lebensfähigkeit der Zellen sowohl in 293mitoPARP als auch in und HeLa S3-Zellen. Um daher als Vorstufe zu dienen, muss extrazelluläres NAD⁺ zu NMN abgebaut werden. Der Zusatz von CMP (kompetitiert mit NMN als Substrat für die Dephosphorylierung durch die externe 5'-Nukleotidase) verringert die Zellviabilität von 293mitoPARP- und HeLa S3-Zellen deutlich. Weiters verlor NMN seine Funktion als extrazellulärer NAD⁺- Vorläufer auch in Gegenwart von Dipyridamol und NBTI, beides Inhibitoren des Plasmamembran-Nukleosid-Transporters. Die mitochondriale NAD⁺-Generierung von NAMN ist auch sensitiv gegenüber diesen Inhibitoren, wenn auch weniger stark (Fig. 4A). Diese Hemmung könnte durch eine Erhöhung der Konzentration von NAMN überwunden werden.

NR unterstützt die mitochondriale PAR-Bildung und Zelllebensfähigkeit in Gegenwart von CMP, während Dipyridamol und NBTI die NR-Nutzung hemmen (Fig. 4B). Aus diesen Ergebnissen kann daher geschlossen werden, dass neben NA und Nam, nur die Ribosid-Vorläufer NR und NAR als extrazelluläre Vorläufer des intrazellulären NAD⁺ dienen, während Mono-(NMN und NAMN) und Dinukleotide (NAD⁺ und NAAD) zunächst in die entsprechenden Nukleoside verarbeitet werden müssen.

### Alle NAD⁺-Biosyntheseenzyme lokalisieren im Zytoplasma oder Nukleus, mit Ausnahme einer mitochondrialen NMNAT Isoform

Zum Verständnis der intrazellulären Signalwege der NAD⁺-Biosynthese wurden umfassende Analysen der subzellulären Verteilung der menschlichen Enzyme durchgeführt. Wenn diese Enzyme mit einem C-terminalen FLAG-Epitop in HeLa S3-Zellen exprimiert werden, lokalisieren alle Enzyme, ausgenommen NMNAT3, im Zytoplasma oder im Zellkern (Fig. 5A). NMNAT3 kolokalisiert mit Mitotracker, für die beiden anderen NMNAT Isoformen ist bekannt, dass sie an der nuklearen bzw. an der zytosolischen Seite des Golgi-Apparates lokalisieren.

Sowohl NamPRT als auch NAPRT erzeugen Substrate für NMNATs. Wenn diese in der vorliegenden Matrix vorhanden wären, könnten sie zur mitochondrialen NAD⁺-Synthese beitragen, vorausgesetzt NMNAT3 ist ein Matrix-Protein. Um diese Frage zu klären wurde der PARAPLAY-Assay angewendet, welcher speziell geeignet ist, suborganellare Proteinlokalisation aufzulösen (Dölle et al., 2010). Dieses Verfahren beinhaltet die Überexpression eines Analyt-Proteins, welches mit PARP1cd fusioniert ist. Für mitochondriale Proteine wird PAR-Akkumulation nur beobachtet, wenn sich das Fusionsprotein innerhalb der Matrix befindet. Daher wurden NMNAT3, NamPRT und NAPRT als Fusionsproteine mit PARP1cd exprimiert. Die Lokalisation dieser Konstrukte (Fig. 5B) war analog zu den entsprechenden FLAG-Proteinen (Fig. 5A): Das NMNAT3-PARP1cd-Protein unterstützt PAR-Akkumulation in Mitochondrien, wodurch ebenfalls belegt wird, dass NMNAT3 in der Tat eine Matrix-Protein ist. Weder das NamPRT- noch das NAPRT-PARP1cd-Fusionsprotein ergab eine nachweisbare Polymerbildung, wodurch belegt wird, dass diese keine Proteine der mitochondrialen Matrix in menschlichen Zellen sind.

Daraus folgt, dass NMNAT3 das einzige Enzym der NAD⁺-Synthese ist, welches in Mitochondrien menschlicher Zellen vorhanden ist. Alle anderen enzymatischen Aktivitäten der menschlichen NAD⁺-Biosynthese liegen im Kern und/oder im Zytoplasma. Daher ist klar, dass nukleares/zytosolisches NAD⁺ aus NR als extrazellulärer Vorstufe synthetisiert werden kann.

### NMN ist die zytosolische Vorstufe der mitochondrialen NAD⁺-Synthese

Die Anwesenheit von nur NMNAT3 in der Matrix weist stark auf NMN als zytosolischen Vorläufer des mitochondrialen NAD⁺ hin. Die Abwesenheit der NAD-Synthetase (NADS) in Mitochondrien schließt die Möglichkeit aus, NA-Vorstufen innerhalb der Organellen zu amidieren. Darüber hinaus deutet die Lokalisation beider NRK Isoformen außerhalb Mitochondrien darauf hin, dass die Phosphorylierung von NR nicht innerhalb der Organellen auftritt.

Nach dem Eintritt in die Zelle, muss NR im Zytosol in NMN werden. In der Tat führt Überexpression von NRK1 (welches zytoplasmatisch ist) zu einer dramatischen Erhöhung der Menge an mitochondrialem PAR, aber nur, wenn ein extrazellulärer Vorläufer (wie NMN) zur Verfügung stand. Somit ist NR ein potenter Vorläufer der mitochondrialen NAD⁺, wenn dies im Zytosol an NMN phosphoryliert wird. Demgemäß wurde hiermit NMN funktionell als der zytosolische Vorläufer von mitochondrialem NAD⁺ bestätigt. Die Überexpression von NRK1 verbesserte erheblich die Verwendung von extrazellulärem NAD⁺ oder NR selbst zur mitochondrialen PAR-Bildung (Fig. 6). Diese Beobachtungen belegen, dass extrazelluläres NMN und NAD⁺ nicht in die Zellen aufgenommen werden, sondern vielmehr zunächst zu NR abgebaut werden müssen.

Dies erklärt auch die besonders schnelle Wirkung von NR im Rahmen der vorliegenden Erfindung bei der synergistischen Wirkung mit EGCG bei der Aktivierung dieser zellulären Prozesse.

## Patentansprüche

1. Getränk, enthaltend jeweils unabhängig voneinander pro 1
- 1 mg bis 10 g, vorzugsweise 1 bis 1000 mg, noch bevorzugter 10 bis 500 mg, insbesondere 50 bis 300 mg, Epigallokatechingallat (EGCG),
- 1 bis 500 mg, vorzugsweise 10 bis 100 mg, insbesondere 20 bis 80 mg, Nikotinamid-Ribose (NR) und/oder 0,05 bis 10 g, vorzugsweise 0,1 bis 5 g, insbesondere 0,5 bis 3 g, Tryptophan.

2. Getränk nach Anspruch 1, **dadurch gekennzeichnet, dass** es zusätzlich Steviosid enthält, vorzugsweise 0,1 bis 500 mg, noch bevorzugter 0,5 bis 200 mg, insbesondere 1 bis 100 mg, Steviosid.

3. Getränk nach Anspruch 1 oder 2, **dadurch gekennzeichnet, dass** es in einem Volumen von 100 ml bis 2 1, vorzugsweise von 250 ml bis 1 1, insbesondere von 330 bis 600 ml vorliegt.

4. Getränkebasis für ein Getränk nach einem der Ansprüche 1 bis 3, **dadurch gekennzeichnet, dass** aus ihr ein Getränk nach einem der Ansprüche 1 bis 3 durch Zugabe von Flüssigkeit, vorzugsweise Wasser oder Fruchtsaft, insbesondere Mineralwasser, hergestellt werden kann.

5. Getränkebasis nach Anspruch 4, **dadurch gekennzeichnet, dass** sie in fester Form, insbesondere in Pulverform, vorliegt.

6. Pharmazeutisches Präparat in Dosiseinheitsform, enthaltend
- 1 mg bis 10 g, vorzugsweise 1 bis 1000 mg, noch bevorzugter 10 bis 500 mg, insbesondere 50 bis 300 mg, Epigallokatechingallat (EGCG),
- 1 bis 500 mg, vorzugsweise 10 bis 100 mg, insbesondere 20 bis 80 mg, Nikotinamid-Ribose (NR) und/oder 0,05 bis 10 g, vorzugsweise 0,1 bis 5 g, insbesondere 0,5 bis 3 g, Tryptophan.

7. Konzentrationssteigerndes Mittel, ausgewählt aus einem Getränk nach einem der Ansprüche 1 bis 3 oder einem pharmazeutischen Präparat nach Anspruch 6.

8. Kombinationspräparat, umfassend EGCG und NR als Wirksubstanzen, insbesondere zur Verwendung als Mittel zur Konzentrationssteigerung.

9. Kombinationspräparat nach Anspruch 8, **dadurch gekennzeichnet, dass** es 10 mg bis 10 g EGCG und 1 bis 100 mg NR, vorzugsweise 100 mg bis 5 g EGCG und 2 bis 50 mg NR, insbesondere 500 mg bis 4 g EGCG und 5 bis 40 g NR, umfasst.

10. Verwendung eines Kombinationspräparats, umfassend EGCG und NR als Wirksubstanzen, als Mittel zur Konzentrationssteigerung.

11. Verwendung nach Anspruch 10, **dadurch gekennzeichnet, dass** das Kombinationspräparat als Getränk oder Getränkebasis nach einem der Ansprüche 1 bis 5.

12. Kombinationspräparat, umfassend EGCG und NR und/oder Tryptophan als Wirksubstanzen, insbesondere zur Verwendung als Mittel zur Konzentrationssteigerung, wobei das Kombinationspräparat aus mindestens zwei getrennt voneinander einnehmbaren Komponenten besteht und in der einen Komponente EGCG und in der anderen Komponente NR und/oder Tryptophan vorgesehen wird.

## Claims

1. A beverage containing, respectively independently of each other, per L:
- 1 mg to 10 g, preferably 1 to 1000 mg, more preferably 10 to 500 mg, in particular 50 to 300 mg, of epigallocatechin gallate (EGCG);
- 1 to 500 mg, preferably 10 to 100 mg, in particular 20 to 80 mg, of nicotinamide ribose (NR) and/or 0.05 to 10 g, preferably 0.1 to 5 g, in particular 0.5 to 3 g of tryptophan.

2. The beverage as claimed in claim 1, **characterized in that** in addition, it contains stevioside, preferably 0.1 to 500 mg, more preferably 0.5 to 200 mg, in particular 1 to 100 mg of stevioside.

3. The beverage as claimed in claim 1 or claim 2, **characterized in that** it is in a volume of 100 mL to 2 L, preferably 250 mL to 1 L, in particular 330 to 600 mL.

4. A beverage base for a beverage as claimed in one of claims 1 to 3, **characterized in that** a beverage as claimed in one of claims 1 to 3 can be produced from it by adding liquid, preferably water or fruit juice, in particular mineral water.

5. The beverage base as claimed in claim 4, **characterized in that** it is in solid form, in particular in the powder form.

6. A pharmaceutical preparation in the form of a dosage unit, containing:
- 1 mg to 10 g, preferably 1 to 1000 mg, more preferably 10 to 500 mg, in particular 50 to 300 mg, of epigallocatechin gallate (EGCG);
- 1 to 500 mg, preferably 10 to 100 mg, in particular 20 to 80 mg, of nicotinamide ribose (NR) and/or 0.05 to 10 g, preferably 0-1 to 5 g, in particular 0.5 to 3 g of tryptophan.

7. concentration-enhancing means selected from a beverage as claimed in one of claims 1 to 3 or a pharmaceutical preparation as claimed in claim 6.

8. A combination preparation comprising EGCG and NR as active substances, in particular for use as a concentration-enhancing means.

9. A combination preparation as claimed in claim 8, **characterized in that** it comprises 10 mg to 10 g of EGCG and 1 to 100 mg of NR, preferably 100 mg to 5 g of EGCG and 2 to 50 mg of NR, in particular 500 mg to 4 g of EGCG and 5 to 40 g of NR.

10. Use of a combination preparation comprising EGCG and NR as active ingredients, as a concentration-enhancing means.

11. Use as claimed in claim 10, **characterized in that** the combination preparation is a beverage or a beverage base as claimed in one of claims 1 to 5.

12. A combination preparation comprising EGCG and NR and/or tryptophan as active substances, in particular for use as concentration-enhancing means, wherein the combination preparation consists of at least two components which can be administered separately, and in that one component is provided with EGCG and the other component is provided with NR and/or tryptophan.

## Revendications

1. Boisson, contenant respectivement indépendamment les uns des autres par 1
- 1 mg à 10 g, de préférence 1 à 1000 mg, de manière encore davantage préférée 10 à 500 mg, notamment 50 à 300 mg, de gallate d'épigallocatéchine (EGCG),
- 1 à 500 mg, de préférence 10 à 100 mg, notamment 20 à 80 mg, de nicotinamide-ribose (NR) et/ou 0,05 à 10 g, de préférence 0,1 à 5 g, notamment 0,5 à 3 g, de tryptophane.

2. Boisson selon la revendication 1, **caractérisée en ce qu'**elle contient en outre du stévioside, de préférence 0,1 à 500 mg, de manière encore davantage préférée 0,5 à 200 mg, notamment 1 à 100 mg, de stévioside.

3. Boisson selon la revendication 1 ou 2, **caractérisée en ce qu'**elle se présente en un volume de 100 ml à 21, de préférence de 250 ml à 11, notamment de 330 à 600 ml.

4. Base pour boisson pour une boisson selon l'une quelconque des revendications 1 à 3, **caractérisée en ce qu'**une boisson selon l'une quelconque des revendications 1 à peut être fabriquée à partir de celle-ci par ajout d'un liquide, de préférence d'eau ou de jus de fruit, notamment d'eau minérale.

5. Base pour boisson selon la revendication 4, **caractérisée en ce qu'**elle se présente sous forme solide, notamment sous forme de poudre.

6. Préparation pharmaceutique sous la forme d'une unité de dose, contenant ;
- 1 mg à 10 g, de préférence 1 à 1 000 mg, de manière encore davantage préférée 10 à 500 mg, notamment 50 à 300 mg, de gallate d'épigallocatéchine (EGCG),
- 1 à 500 mg, de préférence 10 à 100 mg, notamment 20 à 80 mg, de nicotinamide-ribose (NR) et/ou 0,05 à 10 g, de préférence 0,1 à 5 g, notamment 0,5 à 3 g, de tryptophane.

7. Agent augmentant la concentration, choisi parmi une boisson selon l'une quelconque des revendications 1 à à 3 ou une préparation pharmaceutique selon la revendication 6.

8. Préparation combinée, comprenant de l'EGCG et du NR en tant que substances actives, notamment pour une utilisation en tant qu'agent d'augmentation de la concentration.

9. Préparation combinée selon la revendication 8, **caractérisée en ce qu'**elle comprend 10 mg à 10 g d'EGCG et 1 à 100 mg de NR, de préférence 100 mg à 5 g d'EGCG et 2 à 50 mg de NR, notamment 500 mg à 4 g d'EGCG et 5 à 40 g de NR.

10. Utilisation d'une préparation combinée comprenant de l'EGCG et du NR en tant que substances actives, en tant qu'agent pour l'augmentation de la concentration.

11. Utilisation selon la revendication 10, **caractérisée en ce que** la préparation combinée se présente sous la forme d'une boisson ou d'une base pour boisson selon l'une quelconque des revendications 1 à 5.

12. Préparation combinée, comprenant de l'EGCG et du NR et/ou du tryptophane en tant que substances actives, notamment pour une utilisation en tant qu'agent d'augmentation de la concentration, dans lequel la préparation combinée est constituée d'au moins deux composants qui peuvent être pris séparément l'un de l'autre, et de l'EGCG est prévu dans un composant et du NR et/ou du tryptophane sont prévus dans l'autre composant.
